# EUROPEAN PATENT APPLICATION

(11) **EP 4 406 476 A1**
(43) Date of publication of application: **31.07.2024**
(21) Application number: 23386005.5
(22) Date of filing: 27.01.2023
(51) Int. Cl.: A61B 5/024, A63F 13/212, A63F 13/42, A63F 13/65

(54) **DATA PROCESSING APPARATUSES AND METHODS**

(71) Applicant: Sony Interactive Entertainment Inc., Tokyo 108-0075 (JP)
(72) Inventor: Michailidis, Lazaros, London, W1F 7LP (GB)
(74) Representative: D Young & Co LLP

(57) **Abstract**

A data processing apparatus comprising circuitry configured to: receive a signal indicating a simulated cardiogram of a character in a video game, the simulated cardiogram being generated using one or more attributes of the character; determine one or more heart rate variability, HRV, parameters of the character using the simulated cardiogram; and control an occurrence in the video game using the one or more HRV parameters.

## Description

### BACKGROUND

### Field of the Disclosure

This disclosure relates to a data processing apparatuses and methods.

### Description of the Related Art

The "background" description provided is for the purpose of generally presenting the context of the disclosure. Work of the presently named inventors, to the extent it is described in the background section, as well as aspects of the description which may not otherwise qualify as prior art at the time of filing, are neither expressly or impliedly admitted as prior art against the present disclosure.

Video games can sometimes be enhanced by enabling information generated during execution of a game using a first data processing apparatus (e.g. a first games console or personal computer (PC)) to be shared with a second data processing apparatus (e.g. a server).

For example, part of the functionality of a game executed using a games console may be implemented by a server connected to the games console. For instance, this may allow certain features of the game to be updated in real time depending on current events and/or allow a plurality of users to play a game together from remote locations (with each user running the game on their own respective games consoles each of which is connected to the server). In this case, information associated with the game, such as real time data associated with the location of a player's avatar on a gaming map, is transmitted to the server from the games console(s).

It is desirable to allow more information to be transmitted in this way to provide a richer gaming experience. However, transmitting more information requires more network resources. Sharing more information may also present issues regarding ensuring the security of that information.

### SUMMARY

The present disclosure is defined by the claims.

### BRIEF DESCRIPTION OF THE DRAWINGS

Non-limiting embodiments and advantages of the present disclosure are explained with reference to the following detailed description taken in conjunction with the accompanying drawings, wherein:
Fig. 1 schematically shows an example entertainment system;
Figs. 2A and 2B schematically show example components associated with the entertainment system;
Fig. 3 schematically shows an example simulated cardiogram;
Fig. 4 schematically shows a portion of the example simulated cardiogram;
Fig. 5 schematically shows an example technique for generating the simulated cardiogram.
Fig. 6 schematically shows example training data;
Figs. 7A to 7C reproduce Tables 1, 2 and 3 of [1];
Fig. 8 shows a first example method / process; and
Figs. 9A and 9B show a second and third example methods / processes.

Like reference numerals designate identical or corresponding parts throughout the drawings.

### DETAILED DESCRIPTION OF THE EMBODIMENTS

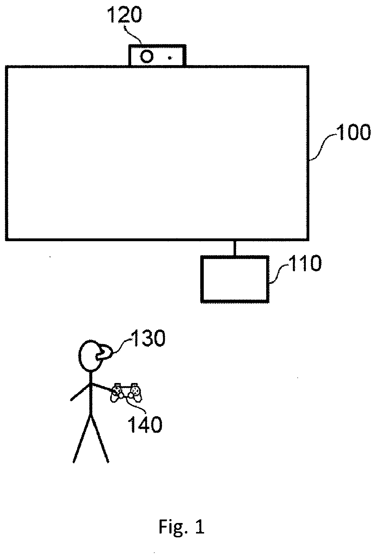

Fig. 1 schematically illustrates an entertainment system suitable for implementing one or more of the embodiments of the present disclosure. Any suitable combination of devices and peripherals may be used to implement embodiments of the present disclosure, rather than being limited only to the configuration shown.

A display device 100 (e.g. a television or monitor), associated with a games console 110, is used to display content to one or more users. A user is someone who interacts with the displayed content, such as a player of a game, or, at least, someone who views the displayed content. A user who views the displayed content without interacting with it may be referred to as a viewer. This content may be a video game, for example, or any other content such as a movie or any other video content. The games console 110 is an example of a content providing device or entertainment device; alternative, or additional, devices may include computers, mobile phones, set-top boxes, and physical media playback devices, for example. In some embodiments the content may be obtained by the display device itself - for instance, via a network connection or a local hard drive.

One or more video and/or audio capture devices (such as the integrated camera and microphone 120) may be provided to capture images and/or audio in the environment of the display device. While shown as a separate unit in Fig. 1, it is considered that such devices may be integrated within one or more other units (such as the display device 100 or the games console 110 in Fig. 1).

In some implementations, an additional or alternative display device such as a head-mountable display (HMD) 130 may be provided. Such a display can be worn on the head of a user, and is operable to provide augmented reality or virtual reality content to a user via a near-eye display screen. A user may be further provided with a video game controller 140 which enables the user to interact with the games console 110. This may be through the provision of buttons, motion sensors, cameras, microphones, and/or any other suitable method of detecting an input from or action by a user.

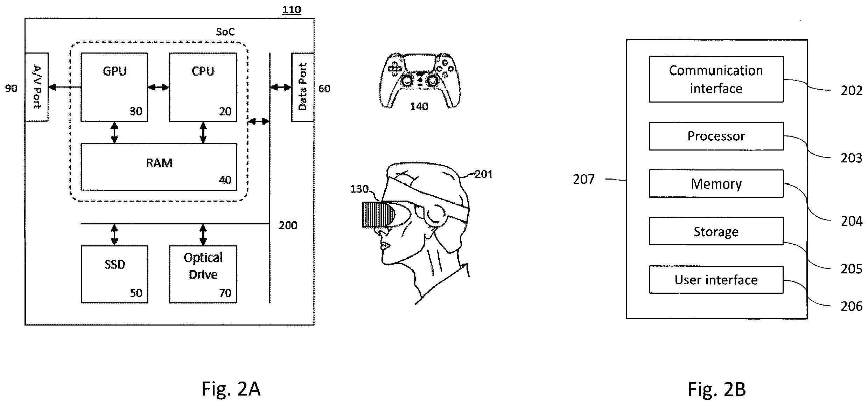

Fig. 2A shows an example of the games console 110. An example is the Sony ^{®} PlayStation 5 ^{®} (PS5). The games console 110 is an example of a data processing apparatus.

The games console 110 comprises a central processing unit or CPU 20. This may be a single or multi core processor, for example comprising eight cores as in the PS5. The games console also comprises a graphical processing unit or GPU 30. The GPU can be physically separate to the CPU, or integrated with the CPU as a system on a chip (SoC) as in the PS5.

The games console also comprises random access memory, RAM 40, and may either have separate RAM for each of the CPU and GPU, or shared RAM as in the PS5. The or each RAM can be physically separate, or integrated as part of an SoC as in the PS5. Further storage is provided by a disk 50, either as an external or internal hard drive, or as an external solid state drive (SSD), or an internal SSD as in the PS5.

The games console may transmit or receive data via one or more data ports 60, such as a universal serial bus (USB) port, Ethernet ^{®} port, WiFi ^{®} port, Bluetooth ^{®} port or similar, as appropriate. It may also optionally receive data via an optical drive 70.

Interaction with the games console is typically provided using one or more instances of the controller 140, such as the DualSense ^{®} handheld controller in the case of the PS5. In an example, communication between each controller 140 and the games console 110 occurs via the data port(s) 60.

Audio/visual (A/V) outputs from the games console are typically provided through one or more A/V ports 90, or through one or more of the wired or wireless data ports 60. The A/V port(s) 90 may also receive audio/visual signals output by the integrated camera and microphone 120, for example. The microphone is optional and/or may be separate to the camera. Thus, the integrated camera and microphone 120 may instead be a camera only. The camera may capture still and/or video images.

Where components are not integrated, they may be connected as appropriate either by a dedicated data link or via a bus 200.

As explained, examples of a device for displaying images output by the game console 110 are the display device 100 and the HMD 130. The HMD is worn by a user 201. In an example, communication between the display device 100 and the games console 110 occurs via the A/V port(s) 90 and communication between the HMD 130 and the games console 110 occurs via the data port(s) 60.

The controller 140 is an example of a peripheral device for allowing the games console 110 to receive input from and/or provide output to the user. Examples of other peripheral devices include wearable devices (such as smartwatches, fitness trackers and the like), microphones (for receiving speech input from the user) and headphones (for outputting audible sounds to the user).

Fig. 2B shows an example of a server 207 (which is another example of a data processing apparatus). It comprises a communication interface 202 for sending electronic information to and/or receiving electronic information from the games console 110, a processor 203 for executing electronic instructions, a memory 204 for storing the electronic instructions to be executed and electronic input and output information associated with the electronic instructions, a storage medium 205 (e.g. a hard disk drive or solid state drive) for long term storage of information and a user interface 206 (e.g. a touch screen, a non-touch screen, buttons, a keyboard and/or a mouse) for receiving commands from and/or outputting information to a user. Each of the communication interface 202, processor 203, memory 204, storage medium 205 and user interface 206 are implemented using appropriate circuitry, for example. The processor 203 controls the operation of each of the communication interface 202, memory 204, storage medium 205 and user interface 206.

Video game characters (such as avatars, these being video game characters controlled by a user in a virtual word) are often designed with different attributes (otherwise referred to as "stats") that provide a layer of information regarding their capabilities. These can include the character's mental state, cognitive workload, fatigue, physical state, physical ability, stamina, circadian changes and sleep stage, for example. Information indicating these attributes may be transmitted from, for example, the games console 110 used to play the video game to the server 207 (e.g. via the data port(s) 60 and communication interface 202) to enable non-player characters (e.g. artificial intelligence, Al, controlled characters) and/or player-owned characters (that is, characters controlled by other players) in the same virtual world to provide appropriate responses. This is applicable, for example, to multiplayer games in which a plurality of users each execute the same game on different respective games consoles 110 and interact with each other in the game by transmitting information to and receiving information from the server 207.

For example, a player's avatar whose stats indicate they are highly stressed may prompt a non-player character in the vicinity to show them a secret door that helps the player's avatar escape a threat. In another example, a player's avatar whose stats indicate they are very relaxed may instigate an in-game event where the avatar is surrounded by enemies forcing them into combat. This helps create a more engaging gaming experience.

Transmitting large amounts of real time avatar stat information to the server, however, requires increased bandwidth resources. It also shares potentially sensitive information about the user and/or their avatar which the user may not want to be shared. For instance, it may reveal information about the history and intention of the user and/or their avatar (e.g. the avatar's location in the virtual world, the recent actions they have taken and the recent characters they have interacted with) which the user is uncomfortable sharing (e.g. because of fears it may be used by another player to obtain an unfair advantage in the game).

There is therefore a desire to be able to communicate an avatar's state in a more compact and privacy-friendly way.

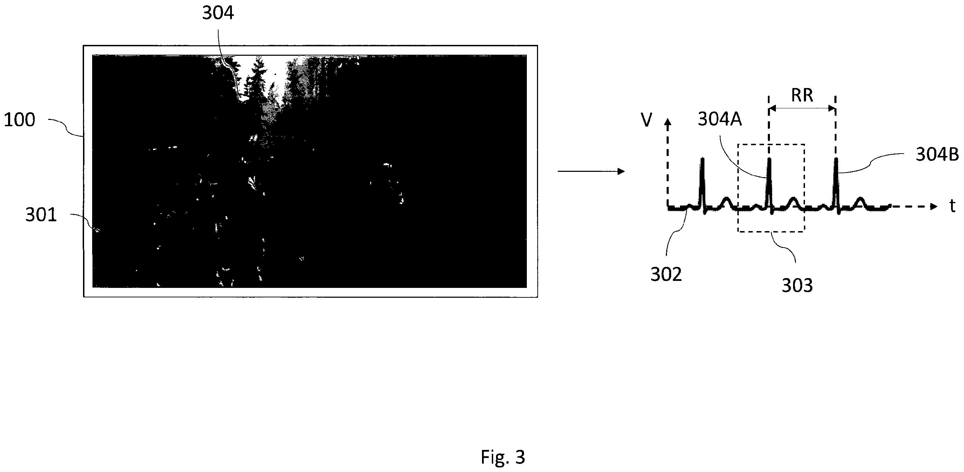

Fig. 3 shows an example technical solution according to the present disclosure. In this example, an interactive gaming image 301 is displayed on the display device 100 which allows a user to control an avatar 304 (e.g. using controller 140). Stats associated with the avatar 304 are used to generate a virtual (or simulated) cardiogram 302. A signal comprising information defining the virtual cardiogram is transmitted to the server 207 instead of transmitting the information of the stats themselves. This helps reduce the amount of data which must be transmitted (especially if a lot of stats data is generated) and helps retain the security of stats data (since it is difficult to obtain the stats data in its original form from the virtual cardiogram). The server 207, however, is able to perform heart rate variability (HRV) analysis on the virtual cardiogram in order to determine information about the avatar which would otherwise only be available from the stats data. The user's gameplay experience may therefore still be enhanced even though the stats data itself is not shared with the server 207.

HRV is the variability in the time intervals between consecutive heartbeats. It can be determined from a cardiogram (which shows a voltage, V, of measured electrical activity of the heart over time, t) by determining the variability in the time interval between consecutive R peaks, for example. This is exemplified with the virtual cardiogram of Fig. 3 (which is designed to mimic a real cardiogram of a real person), which shows two consecutive R peaks 304A and 304B separated by a time interval "RR". The variability in the RR interval between consecutive R peaks of the virtual cardiogram may be measured to determine the HRV.

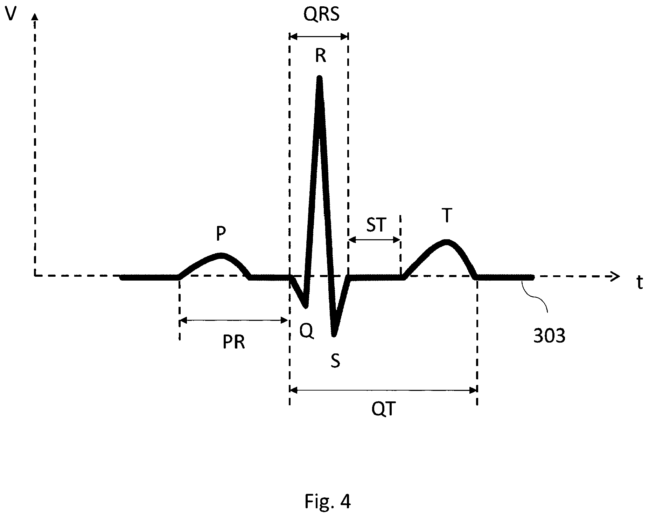

Fig. 4 shows a portion 303 of the virtual cardiogram 302 in more detail. The portion 303 corresponds to one cycle of repeating electrical behaviour of the heart (which, in turn, corresponds to one heartbeat).

The portion 303 comprises a "P" wave (denoted "P" in Fig. 4), a "QRS" complex (defined by points "Q", "R" and "S" in Fig. 4) and a "T" wave (denoted "T" in Fig. 4). Point "R" corresponds to the R peak previously described (R peak 304A of Fig. 3 in this particular case). The electrical activity associated with each heartbeat has these features.

There are a number of measurable time intervals used to represent the temporal relationship between the P wave, QRS complex and T wave. These include the "PR" interval (defining the time between the start of the P wave and the start of the QRS complex), the "QRS" interval (defining the temporal length of the QRS complex), the "QT" interval (defining the time between the start of the QRS complex and the end of the T wave) and the "ST" interval (defining the time between the end of the QRS complex and the start of the T wave).

The relative temporal positions of the P wave, QRS complex and T wave are often different for both the cardiograms of different people and different heartbeats of a cardiogram of a single person. These differences are dependent on many factors including, for example, the physical characteristics of a person (e.g. their age, height and weight) and attributes associated with their behaviour and/or environment (e.g. their current stress level, physical activity level and mental activity level). When such factors are used as stats of an avatar in a video game, it is therefore possible to generate a virtual cardiogram for the avatar based on those stats.

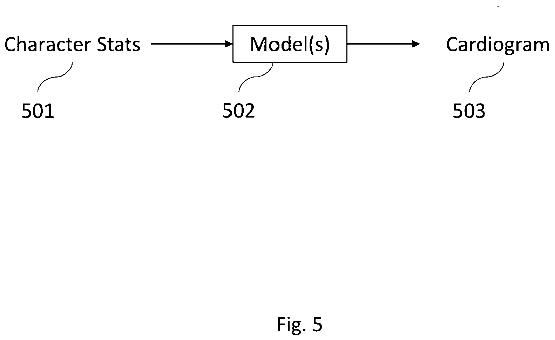

One way of doing this is by using one or more suitably trained machine learning models. This is illustrated in Fig. 5. Here, information 501 indicative of the stats of a character is provided as a set of independent variables to machine learning model(s) 502 which then output information 503 indicative of a virtual cardiogram. This is implemented by the CPU 20 and/or GPU 30 of the games console 110 as part of executed game application, for example.

In one example, as a character is controlled by a user in a game, a set of stats are generated in real time at a rate corresponding to once per heartbeat cycle of the cardiogram. The output information 503 comprises the RR interval between the current heartbeat cycle and the preceding heartbeat cycle and the PR interval, QRS interval, QT interval and ST interval of the current heartbeat cycle. Each new set of stats is then generated after a time equal to the RR interval determined for the previous heartbeat cycle has elapsed and provided to the machine learning model(s) 502 to generate the RR, PR, QRS, QT and ST intervals of the current heartbeat cycle.

Thus, for example, a first set of stats input to the machine learning model(s) 502 at time t₀ generates intervals RR₀, PR₀, QRS₀, QT₀ and ST₀. A second set of stats is then input to the machine learning model(s) 502 at time t₁ (where t₁ = t₀ + RR₀) to generate intervals RR₁, PR₁, QRS₁, QT₁ and ST₁. A third set of stats is then input to the machine learning model(s) 502 at time t₂ (where t₂ = t₁ + RR₁) to generate intervals RR₂, PR₂, QRS₂, QT₂ and ST₂, and so on.

This allows a virtual cardiogram portion (like portion 303) to be generated for each input set of stats and a continuous, real time virtual cardiogram (like virtual cardiogram 302) to be generated by concatenating successively generated cardiogram portions. Thus, for example, the virtual cardiogram will comprise a first portion defined by intervals PR₀, QRS₀, QT₀ and ST₀, followed by a second portion defined by intervals PR₁, QRS₁, QT, and ST₁ and with an R peak separated from the R peak of the first portion by RR₁, followed by a third portion defined by intervals PR₂, QRS₂, QT₂ and ST₂ and with an R peak separated from the R peak of the second portion by RR₂, and so on. In an example, the voltage values of the peak of the P wave, the peak of the T wave and the points Q, R and S are considered to be predetermined fixed respective values. Furthermore, the beginning of the P wave and the end of the T wave of each portion is considered to have a voltage of zero. This allows a smooth and realistic virtual cardiogram to be generated by concatenating the individual virtual cardiogram portions generated from successively collected sets of stats data.

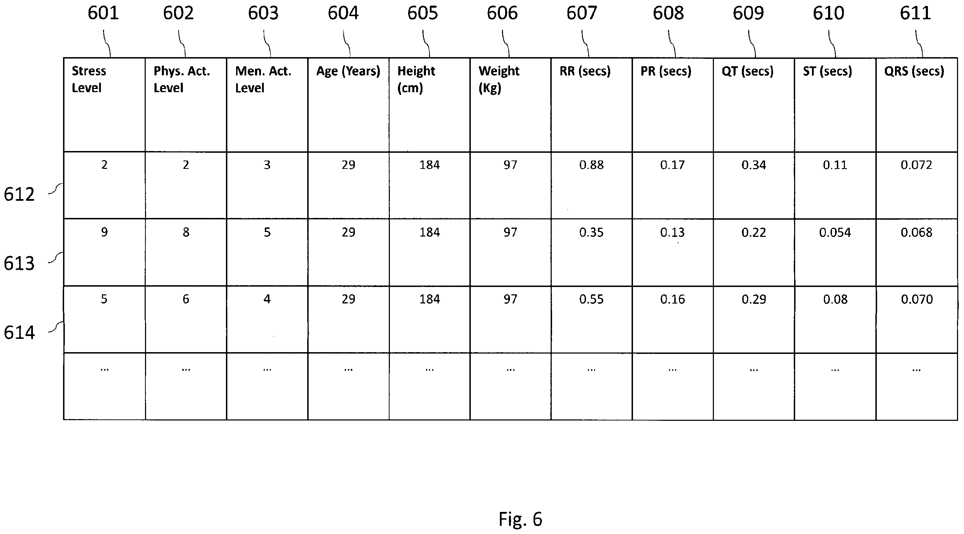

In an example, the RR, PR, QRS, QT and ST intervals are dependent variables generated by different respective machine learning models. Each machine learning model is trained using an appropriate set of training data. Suitable training data is exemplified in Fig. 6.

In Fig. 6, the independent variables for each machine learning model represent different example stats of a gaming character. They are stress level (column 601), physical activity level (column 602), mental activity level (column 603), character age (column 604), character height (column 605) and character weight (column 606).

In this example, stress level ranges from 1 (lowest stress, e.g. when the character is in a location guaranteed to be safe from enemy characters) to 10 (highest stress, e.g. when the character is in combat with the most powerful enemy character possible).

In this example, physical activity level ranges from 1 (lowest physical activity level, e.g. when the character is lying down) to 10 (highest physical activity level, e.g. when the character is running at full speed while jumping over obstacles).

In this example, mental activity level ranges from 1 (lowest mental activity level, e.g. when the character is walking around a safe location and not interacting with any object or any other character) to 10 (highest mental activity level, e.g. when the character has to solve a complex problem in a very short period of time).

In this example, age is in years, height is in centimetres (cm) and weight is in kilograms (Kg).

These stats and their possible values are only examples and, in reality, may depend on the nature of the game being played. In general, the stat(s) and value of each stat for a particular character in a particular game in a particular situation may be determined in advance by the game developer, for example.

Each of the stats is generated by the game being played and has a value which may be variable in real time as, for example, an in-game situation of the gaming character changes (e.g. as the character moves around a virtual map, completes tasks and/or interacts with other gaming characters or, simply, as time goes on).

The value of some stats may have a greater rate of change than others. For example, a character's age may gradually increase (e.g. linearly) as time goes on in the game, their weight may gradually fluctuate (e.g. based on changes to the character's diet and exercise over days, weeks or months in the game) and their height may remain constant. These stats are unlikely to change rapidly with rapid changes in the game (e.g. coming across an enemy or gaining access to a new part of the virtual map). On the other hand, a character's stress, physical activity and mental activity levels may change much more rapidly. For instance, a character simply walking slowly around a peaceful village of the virtual map may experience low levels of stress, physical activity and mental activity. A character exploring a new part of the map in order to work out how to enter a particular building may experience medium levels of stress, physical activity and mental activity. If that character is then set upon by enemy characters and thus has to quickly work out how to enter the building for safety, they may then experience high levels of stress, physical activity and mental activity. The levels of stress, physical activity and mental activity of a character can thus change much more rapidly in the game than that character's age, height or weight.

The values of the stats of the training data are determined, for example, by obtaining real cardiograms of real humans of different ages, heights and weights undertaking various activities associated with different stress, physical activity and mental activity levels.

For example, a random sample of 500 people of the population may be obtained (with their consent) and each person is invited to conduct three different tasks while an electrocardiogram (ECG) of that person is recorded.

The first task is associated with lower levels of stress, physical activity and mental activity level (with the numerical stress, physical activity and mental activity levels of the task being suitably allocated in advance). For example, the first task may be sitting down and watching daytime television while an ECG of the person is recorded. This allows values of the RR, PR, QRS, QT and ST intervals to be determined for the first task (e.g. by determining an average value of RR, PR, QRS, QT and ST over a predetermined period of time such as 10 minutes). The stats and RR, PR, QRS, QT and ST interval data are then associated with each other in the training data, for example as row 612 in Fig. 6.

The second task is associated with higher levels of stress, physical activity and mental activity level (with the numerical stress, physical activity and mental activity levels of the task being suitably allocated in advance). For example, the second task may be running around a locked building to solve a puzzle of how to exit the building within a predetermined period of time (e.g. 10 minutes) while an ECG of the person is recorded. This allows values of the RR, PR, QRS, QT and ST intervals to be determined for the second task (e.g. by determining an average value of RR, PR, QRS, QT and ST over the predetermined period of time). The stats and RR, PR, QRS, QT and ST interval data are then associated with each other in the training data, for example as row 614 in Fig. 6.

The third task is associated with higher levels of stress, physical activity and mental activity level than the first task but lower levels than the second task (with the numerical stress, physical activity and mental activity levels of the task being suitably allocated in advance). For example, the second task may be walking around an unfamiliar neighbourhood with a map to try to get from a point A to a point B while an ECG of the person is recorded. This allows values of the RR, PR, QRS, QT and ST intervals to be determined for the second task (e.g. by determining an average value of RR, PR, QRS, QT and ST over a predetermined period of time such as 10 minutes). The stats and RR, PR, QRS, QT and ST interval data are then associated with each other in the training data, for example as row 613 in Fig. 6.

Optionally, the values of the RR, PR, QRS, QT and ST intervals are initially determined when the person is at rest (e.g. lying down doing nothing), with an average value of RR, PR, QRS, QT and ST being determined over a predetermined period of time (e.g. 10 minutes) while the person is at rest. The at-rest RR, PR, QRS, QT and ST interval values are baseline values (corresponding to resting state HRV) which are then subtracted from the RR, PR, QRS, QT and ST interval values measured during each of the first, second and third tasks. It is then the RR, PR, QRS, QT and ST interval values for each task with the baseline values subtracted which then form the training data. This provides more accurate training data because, for example, it allows innate differences in the subjects (physical activity level, smoking habits or coffee consumption on the day) to be taken into account. If necessary, predetermined baseline RR, PR, QRS, QT and ST interval values may then be added to the output of the trained machine learning model(s) so that the RR, PR, QRS, QT and ST interval values used to generate the virtual cardiogram mimic those of a real person and also take into account the baseline.

It is customary for HRV studies to measure a baseline (resting-state HRV), as there are innate differences between subjects (due to e.g., physical activity level, smoking habits or coffee consumption on the day). If such differences remain unaccounted for, they may distort the effect each task had on the HRV of the subjects. One method to optimize training of a machine learning model is to subtract the derived HRV parameters from the resting baseline. Not sure if this is necessary, but worth mentioning.

For simplicity, Fig. 6 shows an example of such training data for one person. However, in reality, there will be additional rows. For instance, if 500 different people are used and each person completes the first, second and third tasks to generate associated stats and RR, PR, QRS, QT and ST interval data for each task, there will be a total of 1500 rows. In an example, 300 of the rows (representing 20% of the training data) are randomly selected to be a test set, with the remaining 1200 rows forming a training set. The training and test sets are then used to optimise and train the RR, PR, QRS QT and ST machine learning models so that, given a previously unseen combination of input stats generated by a game (e.g. a vector representing stress level, physical activity level, mental activity level, character age, character height and character weight, the values being appropriately pre-processed (e.g. normalised), a value of each of the RR, PR, QRS, QT and ST intervals associated with those input stats may be generated. This allows a virtual cardiogram portion (e.g. like portion 303) to be generated for that set of input stats and a virtual cardiogram to be generated in the way previously described.

In the example of Fig. 6, the machine learning model(s) 502 comprise five machine learning models, one for each of RR, PR, QRS, QT and ST. Once trained, each model takes the gaming stats as input independent variables (e.g. in vector form, as described above) and outputs a respective one of an RR, PR, QRS, QT and ST interval value. Thus, the RR model receives the full set of gaming stats as an input and outputs a value of the RR interval, the PR model receives the full set of gaming stats as an input and outputs a value of the PR interval, and so on. In an example, each machine learning model is a regression model implementing simple or multiple linear regression, polynomial regression, support vector regression (SVR), decision tree regression or random forest regression, for example.

The present technique therefore allows a virtual cardiogram to be generated for a gaming character in real time which mimics the behaviour of the cardiogram of a real human (e.g. of a given age, height and weight) in various situations associated with different stress, physical activity and mental activity levels. Data indicative of the virtual cardiogram may then be transmitted from the games console 110 to the server 207 to allow the server and/or other players to gain information associated with a player's stats without any of those stats actually being transmitted. This is because a virtual cardiogram of a character will be different when that character is in a more stressful, physically intense and/or mentally intense situation than when they are in a less stressful, physically intense and/or mentally intense situation, for example. Less data therefore needs to be transmitted and the security of the stat data itself is maintained. The server 207 (in particular, the processor 203) may use any suitable technique to analyse a received virtual cardiogram to determine information about the gaming character to which the cardiogram relates. In one example, HRV analysis is performed on the virtual cardiogram.

HRV analysis on human cardiograms is able to provide information about a wide variety of attributes. This allows information on, for example, a person's mental state, cognitive workload, fatigue, physical state, physical ability, stamina, circadian changes and sleep stage to be determined. It may also indicate whether a person is likely to be suffering from certain types of diseases or cardiac abnormalities (e.g. cardiovascular disease(s) or arrhythmia). The present technique allows the same HRV analysis to be performed for gaming characters based on a virtual cardiogram generated for that character.

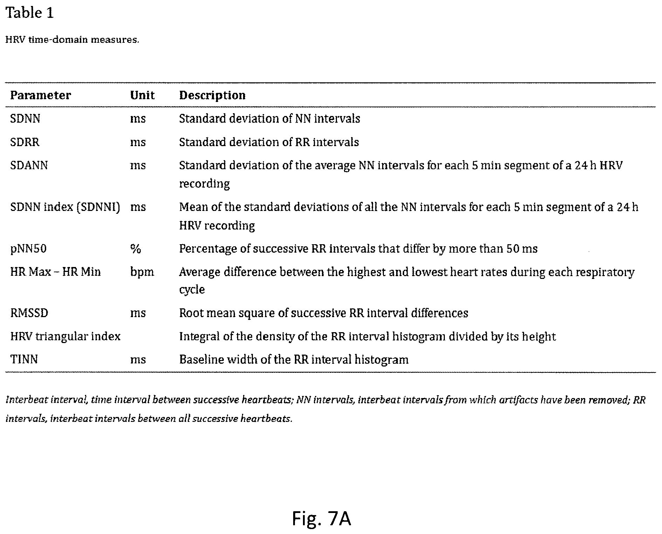

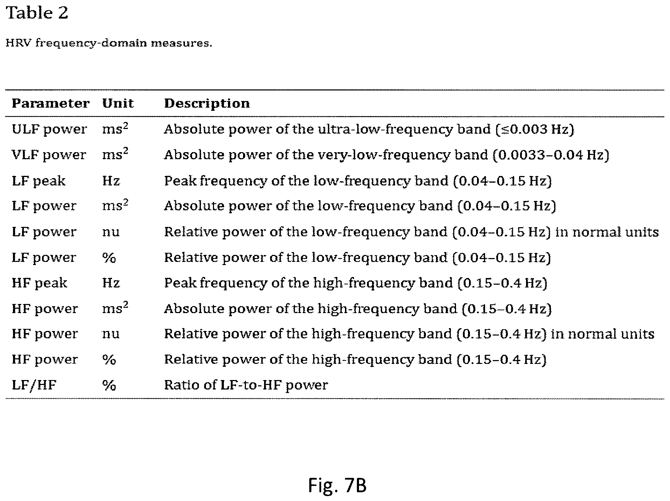

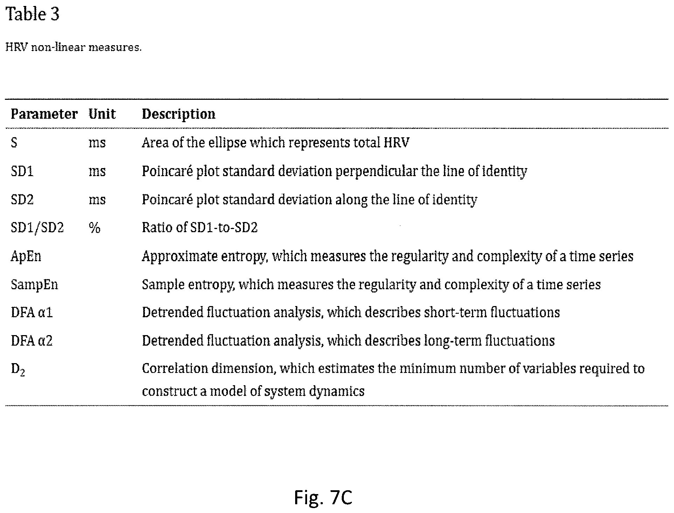

There are many types of HRV analysis of cardiograms including time domain, frequency domain and non-linear measurements. These are discussed in detail in [1], for example. Tables 1, 2 and 3 of [1] are reproduced in Figs. 7A, 7B and 7C, respectively, as examples of HRV parameters which may be determined using virtual cardiograms generated for in-game characters (e.g. as described above).

HRV measurements are made over a certain time period of a cardiogram. For human cardiograms, this may be 24 hours, short term ST (approximately 5 minutes) or ultra-short term UST (less than 5 minutes). For a virtual cardiogram, using ST and UST may be the most appropriate due to the 5 minutes or less time period being applicable to video games (which are played over minutes or a small number of hours rather than 24 hours or more).

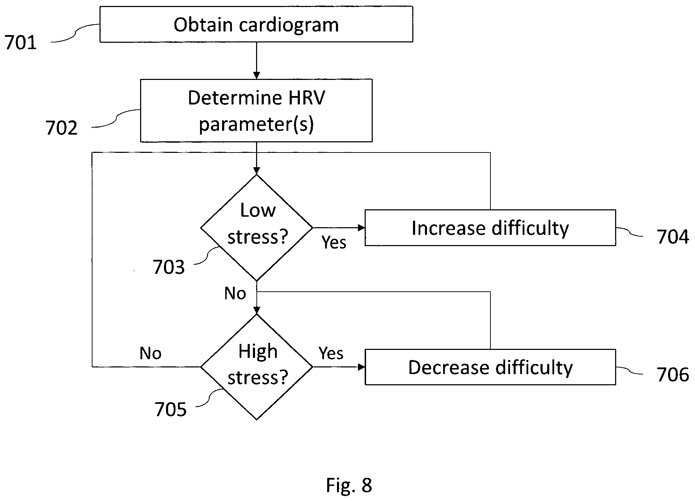

To give an example of the benefits of using HRV analysis for a virtual cardiogram, consider an example in which a gaming character's heart rate is monitored (based on detecting successive RR peaks of its virtual cardiogram) without considering other HRV parameters. In this case, it is difficult to determine whether an increase in heart rate is caused, for instance, by an increase in physical activity (e.g. due to a character starting to run or swim in its virtual environment) or due to an increase in stress of the character (e.g. due to being confronted by enemy characters). However, making measurements of additional HRV parameters, such as RMSSD and pNN50 (which decrease with stress), allows this to be determined more precisely. This is exemplified by the process of Fig. 8. In this example, RMSSD and pNN50 may be determined using a virtual cardiogram generated during the previous X minutes (where X = 5 for ST and X < 5 for UST). The process of Fig. 8 is performed by the server 207 under control of the processor 203, for example. Note that, although RMSSD and pNN50 are used as HRV parameters in this example, this is only an example and the disclosure is not limited to this.

At step 701, a data indicative of a virtual cardiogram of a player's character is obtained. For example, the data is generated by the games console 110 and transmitted to the server 207 via the data port(s) 60 and communication interface 202. In an example, the obtained data represents a virtual cardiogram generated from character stat data collected over the previous X minutes. Data representing an updated virtual cardiogram may be received periodically (e.g. every 5, 10, 20, 30 or 60 seconds) or after a predetermined number of new virtual cardiogram portions 303 (each representing a heartbeat of the character, e.g. every 1, 5 or 10 cardiogram portions) has been generated, for example.

At step 702, one or more HRV parameters are determined using the virtual cardiogram data. For example, RMSSD and pNN50 are determined.

At step 703, it is determined whether the player's character is in a low stress state. For example, if one or both of the values of RMSSD and pNN50 increase above respective first predetermined thresholds (or, for example, increase with a rate of change with a magnitude above respective first predetermined thresholds), it may be determined that the character is in a low stress state.

If the character is in a low stress state, the difficulty of the game is increased at step 704. For example, the server 207 may instigate an in-game event in which the character is surrounded by enemy characters, thereby forcing them into combat. The process then returns to step 703.

If the player's character is not in a low stress state, at step 705, it is determined whether the character is in a high stress state. For example, if one or both of the values of RMSSD and pNN50 decrease below respective second predetermined thresholds (or, for example, decrease with a rate of change with a magnitude above respective second predetermined thresholds), it may be determined that the character is in a high stress state.

If the player's character is in a high stress state, the difficulty of the game is decreased at step 706. For example, the server 207 may cause a non-player character in the vicinity to show the player's character a secret door that helps them escape a threat. The process then returns to step 705.

If the player's character is not in a high stress state, the process returns to step 703.

A more engaging gaming experience may therefore be provided by the server 207 to a player based on stats of that player's gaming character without any of the stat data itself being transmitted to the server. This reduces the amount of data which needs to be transmitted from each games console 110 to the server 207 and helps improve the security of character stat data.

Fig. 8 is only one example of how HRV data may be used and the wide variety of available HRV measurements enables flexibility in how a gaming experience may be made richer based on a virtual cardiogram generated for a player's character. For example, a higher HRV based on conduction abnormalities may be detectable in a virtual cardiogram (as is detectable in human cardiograms). If found, this may increase the susceptibility of a player's character to suffering a cardiac arrest. In this case, a probability of a player's character suffering a cardiac arrest in a given situation may be increased. For instance, the probability may be increased from 0% (for a non-probability HRV analysis) to a non-zero value such as 1%, 2% or 3%. This would mean that, over a given time period (e.g. 1, 2 or 5 minutes), there is a non-zero probability that the player's character suffers a cardiac arrest (and therefore their health bar drops to zero or near-zero). This probability may be increased (e.g. doubled or tripled) in situations of high stress (e.g. as determined at step 705 of Fig. 8).

In an example, additional data indicative of one or more characteristics of the player's character may be provided with the data representing the virtual cardiogram to assist more accurate HRV analysis. For example, age and sex data of the player's character may be provided (since a conclusion based on HRV analysis may depend on such factors, e.g. a particular phenomenon which is normal for men may not be normal for women, or vice versa). This allows, for example, different thresholds (e.g. as used at steps 703 and 705 of Fig. 8) to be used depending on the age and sex data to more accurately determine a character's stress level (and thus to allow gameplay difficulty to be more appropriately dynamically adjusted).

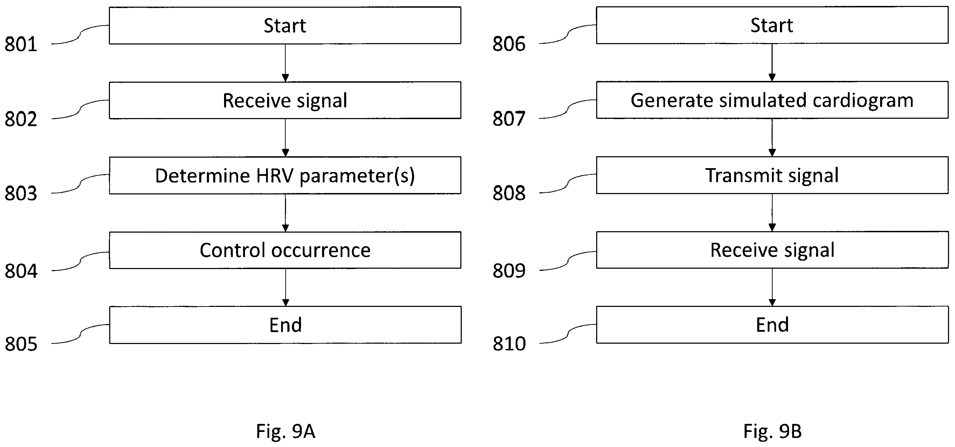

Corresponding data processing methods according to the present technique are shown in Figs. 9A and 9B.

The method of Fig. 9A is a computer-implemented method carried out by circuitry of a data processing apparatus (e.g. the processor 203 of server 207).

The method starts at step 801.

At step 802, a signal indicating a simulated cardiogram (e.g. cardiogram 302) of a character in a video game (e.g. avatar 304) is received. For example, the signal may be received by the server 207 from the games console 110 on which the video game is being played (via data port(s) 60 and communication interface 202). The simulated cardiogram is generated using one or more attributes of the character (e.g. a current stress level, physical activity level, mental activity level, age, height and/or weight of the character).

At step 803, one or more heart rate variability, HRV, parameters of the character are determined using the simulated cardiogram. For example, RMSSD and/or pNN50 may be determined.

At step 804, an occurrence in the video game is controlled using the one or more HRV parameters. For example, the occurrence may be that the character is surrounded by enemy characters if RMSSD and/or pNN50 have recently increased (indicating reduced stress). In another example, the occurrence may be that another character shows the character a secret door to help them escape a threat if RMSSD and/or pNN50 have recently decreased (indicating increased stress). In an example, the processor 203 of the server 207 controls the communication interface 202 to transmit a signal to the data port(s) 60 of the games console 110 to cause the occurrence to be realised in the video game (e.g. by causing a piece of code associated with the occurrence to be executed by the CPU 20 and/or GPU 30).

The method ends at step 805.

The method of Fig. 9B is a computer-implemented method carried out by circuitry of a data processing apparatus (e.g. the CPU 20 and/or GPU 30 of games console 110).

The method starts at step 806.

At step 807, a simulated cardiogram (e.g. cardiogram 302) of a character in a video game (e.g. avatar 304) is generated using one or more attributes of the character (e.g. a current stress level, physical activity level, mental activity level, age, height and/or weight of the character).

At step 808, a signal indicating the simulated cardiogram is transmitted to a second data processing apparatus (e.g. server 207) for determination of one or more heart rate variability, HRV, parameters of the character (e.g. for determination of RMSSD and/or pNN50).

At step 809, a signal is received from the second data processing apparatus indicating an occurrence in the video game controlled using the one or more HRV parameters. For example, the occurrence may be that the character is surrounded by enemy characters if RMSSD and/or pNN50 have recently increased (indicating reduced stress). In another example, the occurrence may be that another character shows the character a secret door to help them escape a threat if RMSSD and/or pNN50 have recently decreased (indicating increased stress). In an example, the processor 203 of the server 207 controls the communication interface 202 to transmit a signal to the data port(s) 60 of the games console 110 to cause the occurrence to be realised in the video game (e.g. by causing a piece of code associated with the occurrence to be executed by the CPU 20 and/or GPU 30).

The method ends at step 810.

Example(s) of the present technique are defined by the following numbered clauses:
1. A data processing apparatus comprising circuitry configured to:
   receive a signal indicating a simulated cardiogram of a character in a video game, the simulated cardiogram being generated using one or more attributes of the character;
   determine one or more heart rate variability, HRV, parameters of the character using the simulated cardiogram; and
   control an occurrence in the video game using the one or more HRV parameters.
2. A data processing apparatus according to clause 1, wherein:
   the one or more HRV parameters comprise one or more of RMSSD and pNN50;
   if a value of RMSSD or pNN50 exceeds a first threshold or a magnitude of a rate of increase of a value of RMSSD or pNN50 exceeds a first threshold, control an occurrence in the video game to increase a difficulty level of the video game; and
   if a value of RMSSD or pNN50 falls below a second threshold or a magnitude of a rate of decrease of a value of RMSSD or pNN50 exceeds a second threshold, control an occurrence in the video game to reduce a difficulty level of the video game
3. A data processing apparatus according to any preceding clause, wherein the circuitry is configured to:
   receive data indicating a characteristic of the character in addition to the signal indicating the simulated cardiogram; and
   control an occurrence in the video game using the one or more HRV parameters and the data indicating the characteristic of the character.
4. A data processing apparatus according to any preceding clause, wherein the circuitry is configured to:
   determine, using the one or more HRV parameters, whether the HRV indicates a heart conduction abnormality; and
   if the HRV indicates a conduction abnormality, control, as an occurrence in the video game, a probabilistic cardiac arrest of the character.
5. A data processing apparatus according to any preceding clause, wherein the one or more HRV parameters are determined over the short term (ST) or ultra-short term (UST).
6. A data processing apparatus comprising circuitry configured to:
   generate a simulated cardiogram of a character in a video game using one or more attributes of the character; and
   transmit a signal indicating the simulated cardiogram to a second data processing apparatus for determination of one or more heart rate variability, HRV, parameters of the character; and
   receive a signal from the second data processing apparatus indicating an occurrence in the video game controlled using the one or more HRV parameters.
7. A data processing apparatus according to clause 6, wherein the simulated cardiogram is generated by inputting a value of the one or more attributes of the character into one or more trained machine learning models to generate one or more values defining a portion of the simulated cardiogram corresponding to one heartbeat of the character.
8. A data processing apparatus according to clause 7, wherein the one or more attributes of the character comprise one or more of a stress level, physical activity level and mental activity level of the character.
9. A data processing apparatus according to clause 7 or 8, wherein the one or more values defining a portion of the simulated cardiogram corresponding to one heartbeat of the character comprise one or more of values of an RR interval, a PR interval, a QT interval, an ST interval and a QRS interval.
10. A data processing apparatus according to any one of clauses 6 to 9, wherein:
   the circuitry is configured to transmit data indicating a characteristic of the character in addition to the signal indicating the simulated cardiogram; and
   the occurrence in the video game indicated by the signal received from second data processing apparatus is controlled using the one or more HRV parameters and the data indicating the characteristic of the character.
11. A method of controlling a data processing apparatus, the method comprising controlling the data processing apparatus to:
   receive a signal indicating a simulated cardiogram of a character in a video game, the simulated cardiogram being generated using one or more attributes of the character;
   determine one or more heart rate variability, HRV, parameters of the character using the simulated cardiogram; and
   control an occurrence in the video game using the one or more HRV parameters.
12. A method of controlling a data processing apparatus, the method comprising controlling the data processing apparatus to:
   generate a simulated cardiogram of a character in a video game using one or more attributes of the character; and
   transmit a signal indicating the simulated cardiogram to a second data processing apparatus for determination of one or more heart rate variability, HRV, parameters of the character; and
   receive a signal from the second data processing apparatus indicating an occurrence in the video game controlled using the one or more HRV parameters.
13. A program for controlling a computer to perform a method according to clause 11 or 12.
14. A storage medium storing a program according to clause 13.
15. A system comprising a first data processing apparatus according to clause 1 and a second data processing apparatus according to clause 6.

Numerous modifications and variations of the present disclosure are possible in light of the above teachings. It is therefore to be understood that, within the scope of the claims, the disclosure may be practiced otherwise than as specifically described herein.

In so far as embodiments of the disclosure have been described as being implemented, at least in part, by one or more software-controlled information processing apparatuses, it will be appreciated that a machine-readable medium (in particular, a non-transitory machine-readable medium) carrying such software, such as an optical disk, a magnetic disk, semiconductor memory or the like, is also considered to represent an embodiment of the present disclosure. In particular, the present disclosure should be understood to include a non-transitory storage medium comprising code components which cause a computer to perform any of the disclosed method(s).

It will be appreciated that the above description for clarity has described embodiments with reference to different functional units, circuitry and/or processors. However, it will be apparent that any suitable distribution of functionality between different functional units, circuitry and/or processors may be used without detracting from the embodiments.

Described embodiments may be implemented in any suitable form including hardware, software, firmware or any combination of these. Described embodiments may optionally be implemented at least partly as computer software running on one or more computer processors (e.g. data processors and/or digital signal processors). The elements and components of any embodiment may be physically, functionally and logically implemented in any suitable way. Indeed, the functionality may be implemented in a single unit, in a plurality of units or as part of other functional units. As such, the disclosed embodiments may be implemented in a single unit or may be physically and functionally distributed between different units, circuitry and/or processors.

Although the present disclosure has been described in connection with some embodiments, it is not intended to be limited to these embodiments. Additionally, although a feature may appear to be described in connection with particular embodiments, one skilled in the art would recognize that various features of the described embodiments may be combined in any manner suitable to implement the present disclosure.

### REFERENCES

[1] Shaffer F, Ginsberg JP. An Overview of Heart Rate Variability Metrics and Norms. Front Public Health. 2017 Sep 28;5:258. doi: 10.3389/fpubh.2017.00258. PMID: 29034226; PMCID: PMC5624990.

## Claims

1. A data processing apparatus comprising circuitry configured to:
receive a signal indicating a simulated cardiogram of a character in a video game, the simulated cardiogram being generated using one or more attributes of the character;
determine one or more heart rate variability, HRV, parameters of the character using the simulated cardiogram; and
control an occurrence in the video game using the one or more HRV parameters.

2. A data processing apparatus according to claim 1, wherein:
the one or more HRV parameters comprise one or more of RMSSD and pNN50;
if a value of RMSSD or pNN50 exceeds a first threshold or a magnitude of a rate of increase of a value of RMSSD or pNN50 exceeds a first threshold, control an occurrence in the video game to increase a difficulty level of the video game; and
if a value of RMSSD or pNN50 falls below a second threshold or a magnitude of a rate of decrease of a value of RMSSD or pNN50 exceeds a second threshold, control an occurrence in the video game to reduce a difficulty level of the video game

3. A data processing apparatus according to any preceding claim, wherein the circuitry is configured to:
receive data indicating a characteristic of the character in addition to the signal indicating the simulated cardiogram; and
control an occurrence in the video game using the one or more HRV parameters and the data indicating the characteristic of the character.

4. A data processing apparatus according to any preceding claim, wherein the circuitry is configured to:
determine, using the one or more HRV parameters, whether the HRV indicates a heart conduction abnormality; and
if the HRV indicates a conduction abnormality, control, as an occurrence in the video game, a probabilistic cardiac arrest of the character.

5. A data processing apparatus according to any preceding claim, wherein the one or more HRV parameters are determined over the short term (ST) or ultra-short term (UST).

6. A data processing apparatus comprising circuitry configured to:
generate a simulated cardiogram of a character in a video game using one or more attributes of the character; and
transmit a signal indicating the simulated cardiogram to a second data processing apparatus for determination of one or more heart rate variability, HRV, parameters of the character; and
receive a signal from the second data processing apparatus indicating an occurrence in the video game controlled using the one or more HRV parameters.

7. A data processing apparatus according to claim 6, wherein the simulated cardiogram is generated by inputting a value of the one or more attributes of the character into one or more trained machine learning models to generate one or more values defining a portion of the simulated cardiogram corresponding to one heartbeat of the character.

8. A data processing apparatus according to claim 7, wherein the one or more attributes of the character comprise one or more of a stress level, physical activity level and mental activity level of the character.

9. A data processing apparatus according to claim 7 or 8, wherein the one or more values defining a portion of the simulated cardiogram corresponding to one heartbeat of the character comprise one or more of values of an RR interval, a PR interval, a QT interval, an ST interval and a QRS interval.

10. A data processing apparatus according to any one of claims 6 to 9, wherein:
the circuitry is configured to transmit data indicating a characteristic of the character in addition to the signal indicating the simulated cardiogram; and
the occurrence in the video game indicated by the signal received from second data processing apparatus is controlled using the one or more HRV parameters and the data indicating the characteristic of the character.

11. A method of controlling a data processing apparatus, the method comprising controlling the data processing apparatus to:
receive a signal indicating a simulated cardiogram of a character in a video game, the simulated cardiogram being generated using one or more attributes of the character;
determine one or more heart rate variability, HRV, parameters of the character using the simulated cardiogram; and
control an occurrence in the video game using the one or more HRV parameters.

12. A method of controlling a data processing apparatus, the method comprising controlling the data processing apparatus to:
generate a simulated cardiogram of a character in a video game using one or more attributes of the character; and
transmit a signal indicating the simulated cardiogram to a second data processing apparatus for determination of one or more heart rate variability, HRV, parameters of the character; and
receive a signal from the second data processing apparatus indicating an occurrence in the video game controlled using the one or more HRV parameters.

13. A program for controlling a computer to perform a method according to claim 11 or 12.

14. A storage medium storing a program according to claim 13.

15. A system comprising a first data processing apparatus according to claim 1 and a second data processing apparatus according to claim 6.
